# EUROPEAN PATENT APPLICATION

(11) **EP 3 217 761 A1**
(43) Date of publication of application: **13.09.2017**
(21) Application number: 17160052.1
(22) Date of filing: 09.03.2017
(51) Int. Cl.: H05B 3/14, H05B 3/34, A61F 7/00

(54) **ELECTRICALLY AND THERMALLY CONDUCTIVE ELECTRODE DEVICE WITH FAR INFRARED RADIATION AND MANUFACTURING METHOD THEREOF**

(30) Priority: 09.03.2016 TW 105107269
(71) Applicant: EASYWELL BIOMEDICALS, INC., Hsinchu City (TW)
(72) Inventor: CHUANG, CHENG-LIN, HSINCHU CITY (TW); HSIEH, CHIH-WEI, HSINCHU CITY (TW)
(74) Representative: Pallini Gervasi, Diego

(57) **Abstract**

A far infrared electrically and thermally conductive electrode device includes an electrode set (20) and a connection assembly (30). The electrode set (20) is used to connect to a host (10) in a wireless way. The electrode set (20) is electrically and thermally conductive. A method for making the electrode of the electrode device includes steps of disposing an electrode protecting layer (21) on a first side of a far infrared heating (FIR) layer (22); disposing an electrode insulating layer (23) on a second side of the FIR layer (22); disposing an electrode layer (24) on the electrode insulating layer (23); and covering the electrode layer (24) with a conducting gel layer (25). The electrode set (20) emits far infrared rays and generates heat to simulates nerves of tissue under a person's skin.

## Description

### 1. Field of the Invention

The invention relates to an electrically and thermally conductive electrode device and, in particular, to a far infrared electrically and thermally conductive electrode device. The invention also relates to a method of the making the electrode thereof.

### 2. Description of Related Art

With progressive advancement in technology, a variety of medical equipment for alleviating pains can be used without the help of professional healthcare personnel. It is already very common to use electrode pads widely sold in the market for electrical nerve stimulation. Through weak AC (Alternating Current) current at different frequencies, shallow epidermal nerves and even the brain can be stimulated so that the brain releases endorphin to alleviate pains.

For example, when using electrode pads for electrotherapy, current at a stimulating frequency between 2Hz and 10Hz has a long-lasting effect in pain release. On the other hand, current at a stimulating frequency between 10Hz and 30Hz can ease muscle swelling and facilitate blood circulation, in addition to the effect of massaging muscle to reduce muscle fatigue. However, human skin has a large resistance against penetration of low-frequency current. Low-frequency electrical stimulations thus have a limited penetrating power, rendering the pain-relieving effects staying at shallow places. Although medium-frequency electrical stimulations (generally defined to be at a frequency range of 1kHz to 10kHz) can go deeper into the muscles (i.e. higher frequencies have a better penetrating power), their pain-relieving effects are not so outstanding. Therefore, a conventional technique called modulated medium-frequency electrotherapy was brought into play for pain relief. Clinic tests indicate that the modulated medium-frequency electrotherapy has a better effect in pain relief.

However, one needs to attach or remove the electrode pads currently available in the market to the skin over and over again, if frequent use is needed. This imposes stringent requirements on the quality of the electrode pads. Even if an electrode pad is reusable, more than one electrode wire may be required to connect a host and the electrode pad. During electrical stimulation, skin functions as a medium to form an electrical loop for electrotherapy. In order for the electrode pad to have uniform and smooth contact with the skin, one usually applies an electrically conductive gel between the electrode pad and the skin. This further allows the electrical current to be more uniformly distributed over the muscular nerves, achieving the effect of electrical stimulation for local pain relief.

It can be seen from the above description of the prior art that the reusable electrode pad needs to have a high quality. Even so, the electrode pads cause operational inconvenience arising from the wired connection. Moreover, existing electrode pads only provide electrical stimulations without relaxing shallow and deeper layers of muscles. It is therefore imperative to provide a better solution that is more convenient for the user and can deeply relaxing skin and muscles.

In view of the foregoing, an objective of the invention is to provide a far infrared (FIR) electrically and thermally conductive electrode device and a method for making the electrode of the electrode device thereof. The invention has an electrode structure that is easy for replacement and has the effects of electrically and thermally conducting as well as emitting FIR rays. The electrode structure emits FIR rays to heat up and electrically conducts to stimulate nerves in different layers of tissue under the skin.

To achieve the foregoing objective, the method includes steps of:
providing an FIR heating layer;
attaching an electrode protecting layer to an upper surface of the FIR heating layer;
attaching an electrode insulating layer to a lower surface of the FIR heating layer;
attaching an electrode layer to a lower surface of the electrode insulating layer; and
covering a lower surface of the electrode layer with a conducting gel layer.

According to the above-mentioned method, the electrode protecting layer and the electrode insulating layer are disposed respectively on the upper and lower surfaces of the FIR heating layer. The electrode layer is disposed on the lower surface of the electrode insulating layer and the lower surface of the electrode layer is covered by a conducting gel layer. Such an electrode structure can simultaneously emit far infrared waves, generate heat, and electrically conduct to achieve the goal of stimulating nerves in different layers of tissue under the skin.

To achieve the objective of the invention, the FIR electrically and thermally conductive electrode device includes a connection assembly, an electrode set and a host.

The connection assembly has at least one electrode connecting end.

The electrode set is connected to the at least one electrode connecting end of the connection assembly, and includes an electrode protecting layer, an FIR heating layer, an electrode insulating layer, an electrode layer, and a conducting gel layer.

The host is connected to the at least one electrode connecting end of the connection assembly and supplies power to the electrode set.

The electrode protecting layer is disposed on an upper surface of the FIR heating layer, the electrode insulating layer is disposed on an lower surface of the FIR heating layer, an upper surface of the electrode layer is disposed on a lower surface of the electrode insulating layer, and the conducting gel layer covers a lower surface of the electrode layer.

With the above-mentioned structure, the electrode set can be connected to the host via the connection assembly in a wireless way. The electrode set is connected to the electrode connecting end of the connection assembly to ease users to make a replacement. The electrode protecting layer is disposed on the upper surface of the FIR heating layer. The electrode insulating layer is disposed on the lower surface of the FIR heating layer. The upper surface of the conducting layer is disposed on the lower surface of the electrode insulating layer. The conducting gel layer covers the lower surface of the conducting layer. When the host supplies power, the electrode set can simultaneously emit FIR rats, generate heat, and electrically conduct to stimulate nerves in different layers of tissue under the skin.
In the drawings
FIG. 1 shows a perspective view of a first embodiment of a far infrared electrically and thermally conductive electrode device in accordance with the invention;
FIG. 2 is a partially exploded perspective view of the electrode device in FIG. 1;
FIG. 3 shows a perspective view of a second embodiment of a far infrared electrically and thermally conductive electrode device in accordance with the invention;
FIG. 4 is an exploded perspective view of the electrode device in FIG. 3; and
FIG. 5 is an enlarged exploded perspective view of the electrode device in FIG. 4.

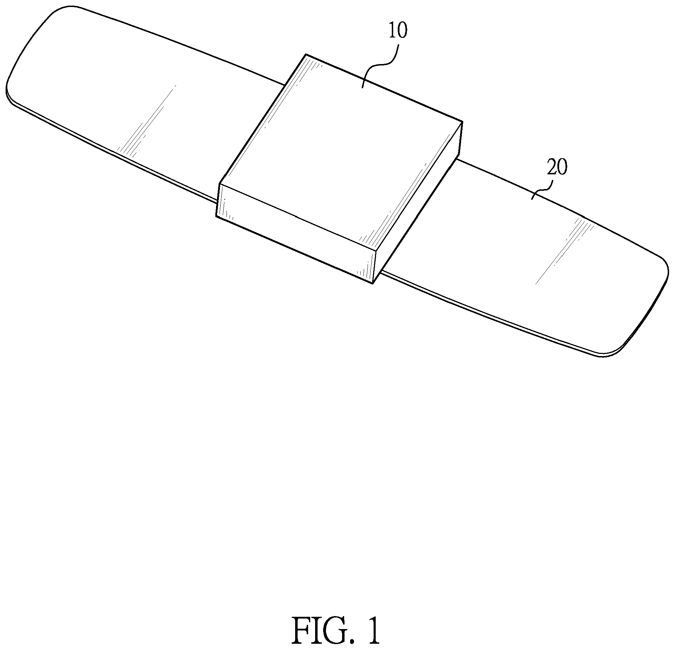

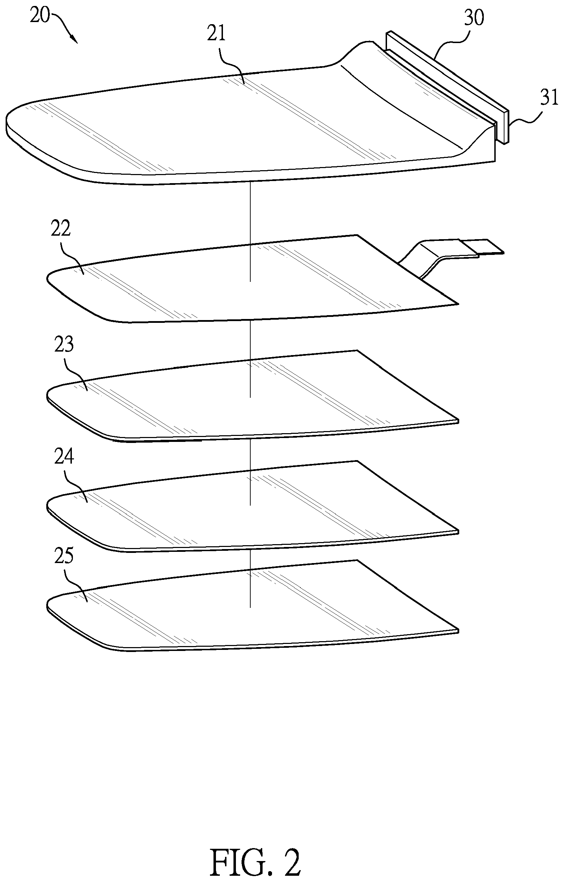

A first embodiment of an FIR (Far Infrared) electrically and thermally conductive electrode device in accordance with the present invention is shown in FIGS. 1 and 2 and includes a host 10, an electrode set 20, and a connection assembly 30. As shown in FIG. 2, the electrode set 20 is electrically connected to at least one electrode connecting end 31 of the connection assembly 30, via which the host 10 is electrically connected. In this embodiment, the electrode set 20 includes one or more electrodes. The size and shape of the electrode set 20 can have different designs according to the skin, the body and contact area thereof.

In this embodiment, the connection assembly 30 and the host 10 are connected in a wireless way using a set of male and female buckle connectors. For example, the connection assembly 30 has a male buckle connector, and the host 10 has a female buckle connector. When the male buckle connection assembly and the female buckle connection assembly are connected, the electrode connecting end 31 is electrically connected to a power loop inside the host 10. As another example, the connection assembly 30 has a female buckle connector, and the host 10 has a male buckle connector. Likewise, when the female buckle connector and the male buckle connector are connected, the electrode connecting end 31 also establishes an electrical connection with the power loop inside the host 10. Furthermore, the set of male and female buckle connectors may be a set of magnetic connectors or a set of magnetic pogo pin connectors. Both have the magnetic and alignment features.

It is noted that FIR light is generally called the light of life and is the most penetrating light into the skin and deep layers of tissue under the skin. Although human cannot see FIR light with naked eyes, the properties of FIR light are similar to those of visible light, which can not only propagate straight along an optical axis but also get reflected and radiate. Therefore, FIR light can be quickly absorbed by human skins. FIR rays entering human body can cause vibration of the atoms and molecules of the tissues. Through resonant absorption, the temperature of the deep layers of tissue under the skin rises. When used on human skin, the electrode set 20 can achieve the effects of expanding blood capillaries and facilitating blood circulation.

As shown in FIG. 2, the electrode set 20 includes an electrode protecting layer 21, an FIR heating layer 22, an electrode insulating layer 23, an electrode layer 24, and a conducting gel layer 25. The electrode protecting layer 21 is attached to an upper surface of the FIR heating layer 22. The electrode insulating layer 23 is attached to a lower surface of the FIR heating layer 22. An upper surface of the electrode layer 24 is disposed on a lower surface of the electrode insulating layer 23. An upper surface of the conducting gel layer 25 covers the lower surface of the electrode layer 24.

Furthermore, the FIR heating layer 22 in this embodiment is prepared by high-temperature carbonization. A polyacrylonitrile carbon fiber (PANCF) is processed at a high temperature (about 1100°C) as a porous carbon fiber heating material, which can emit FIR rays ranging from 4 to 16 microns (µm) in wavelength at a high temperature. The carbon fiber heating material differs from usual metal heating materials in that it does not generate electromagnetic waves. In this embodiment, the electrode layer 24 may be formed by an electrically conductive printing technique. Alternatively, the electrode layer 24 may contain electrically conductive dopants. Moreover, the electrode layer 24 may be printed on or attached to the electrode insulating layer 23. A lower surface of the conducting gel layer 25 is attached to the skin where requires care for pain relief. In this embodiment, the conducting gel layer 25 achieves electrical conduction via the conductive ions therein. The user can selectively replace the gel layer according to the degree of comfort of electrical stimulations.

The disclosed FIR electrically and thermally conductive electrode device can be connected to the host 10 via the connection assembly 30. The electrode set 20 is electrically connected to the electrode connecting end 31 of the connection assembly 30, allowing the user to easily replace the electrode set 20. When the host 10 supplies power to the electrode set 20, the set of electrodes 20 can emit FIR rays, generate heat, and make the electrode set 20 electrically conducting, thereby enhancing stimulation effects on nerves in different layers of tissue under the skin. According to the above-mentioned description of the first embodiment, a method for making the electrode of the FIR electrically and thermally conductive electrode device includes steps of: providing the FIR heating layer 22; attaching the electrode protecting layer 21 to the upper surface of the FIR heating layer 22; attaching the electrode insulating layer 23 to the lower surface of the FIR heating layer 22; providing an electrode layer 24 and attaching the electrode layer 24 to the lower surface of the electrode insulating layer 23; and covering the lower surface of the electrode layer 24 with the conducting gel layer 25.

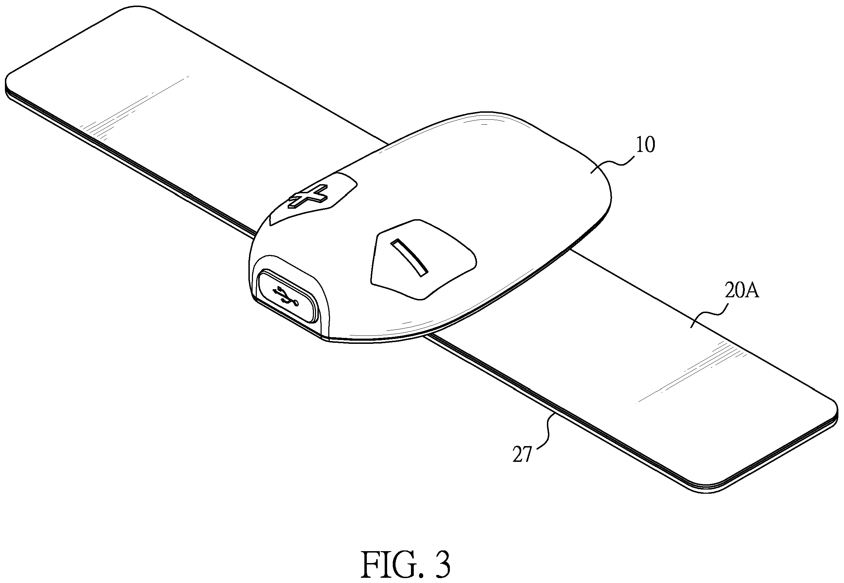

A second embodiment of an FIR electrically and thermally conductive electrode device in accordance with the present invention is shown in FIGS. 3 to 5. The present embodiment differs from the first embodiment in the structures of the electrode set 20 and the connection assembly 30. This embodiment has an electrode set 20A and a connection assembly 30A. The electrode set 20A includes an electrode protecting layer 21 A, an FIR heating unit 26, and a conducting gel layer 25. In this embodiment, the conducting gel layer 25 further includes an insulating layer, a conducting layer 25A, and a gel layer 27.

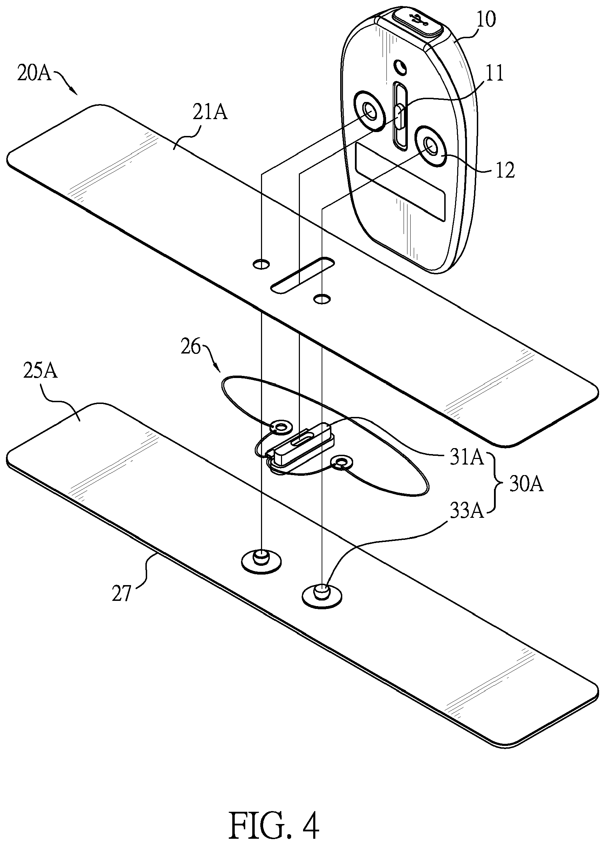

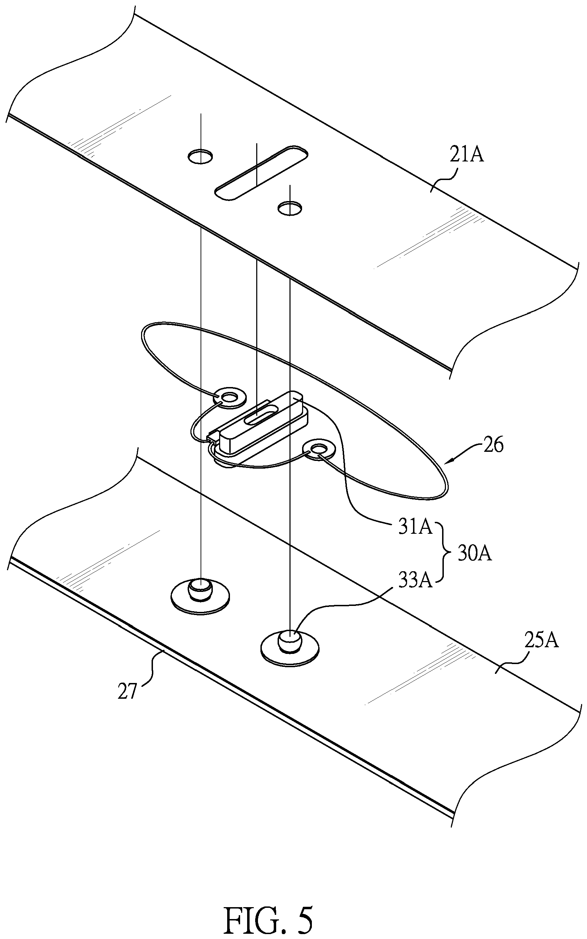

With reference to FIGS. 4 and 5, the connection assembly 30A includes a first connector 31A and a plurality of first positioning members 33A. The first connector 31A is installed on the FIR heating unit 26. The plurality of positioning members 33A are provided on an upper surface of the conducting layer 25A. The electrode protecting layer 21A has a plurality of through holes corresponding to the first connectors 31 A and the first positioning members 33A in size respectively. When intending to detachably connect the electrode set 20A to the host 10 via the connection assembly 30A, the user only needs to let the first positioning members 33A on the conducting layer 25A and the first connector 31A on the FIR heating unit 26 go through the respective through holes on the electrode protecting layer 21A for the connection assembly 30A to be fixed to the bottom of the host 10 in a detachable way.

Furthermore, the host 10 in this embodiment is provided with a second connector 11 and a plurality of second positioning members 12. The second connector 11 on the bottom of the host 10 is connected to the first connector 31A of the FIR heating unit 26, and the second positioning members 12 on the bottom of the host 10 are connected to the respective first positioning members 33A.

The invention being thus described, it will be obvious that the same may be varied in many ways. Such variations are not to be regarded as a departure from the spirit and scope of the invention, and all such modifications as would be obvious to one skilled in the art are intended to be included within the scope of the following claims.

## Claims

1. A method for making a far infrared (FIR) electrically and thermally conductive electrode device, **characterized in that** the method comprises the steps of:
providing an FIR heating layer (22);
attaching an electrode protecting layer (21) to an upper surface of the FIR heating layer(22);
attaching an electrode insulating layer (23) to a lower surface of the FIR heating layer (22);
attaching an electrode layer (24) to a lower surface of the electrode insulating layer(23); and
covering a lower surface of the electrode layer (24) with a conducting gel layer (25).

2. The method of claim 1, wherein the FIR heating layer (22) is made from a porous carbon fiber heating material obtained by processing polyacrylonitrile carbon fibers (PANCF) at a high temperature.

3. The method of claim 2, wherein the carbon fiber heating material emits FIR rays ranging from 4 to 16 microns (µm) in wavelength while the carbon fiber heating material generating a high temperature.

4. The method of claim 1, wherein the electrode layer (24) is formed using an electrically conductive printing technique, or the electrode layer contains electrically conductive dopants.

5. The method of claim 1, wherein the conducting gel layer (25) is electrically conducting by virtue of conductive ions therein.

6. A far infrared (FIR) electrically and thermally conductive electrode device, **characterized in that** the device comprises:
a connection assembly (30) having at least one electrode connecting end (31);
an electrode set (20) connected to the at least one electrode connecting end (31) of the connection assembly (30), and including an electrode protecting layer (21), an FIR heating layer (22), an electrode insulating layer (23), an electrode layer (24), and a conducting gel layer (25); and
wherein the electrode protecting layer (21) is disposed on an upper surface of the FIR heating layer (22), the electrode insulating layer (23) is disposed on an lower surface of the FIR heating layer (22), an upper surface of the electrode layer (24) is disposed on a lower surface of the electrode insulating layer (23), and the conducting gel layer (25) covers a lower surface of the electrode layer (24).

7. The device of claim 6, wherein the electrode set (20) includes one or more electrodes.

8. The device of claim 6, wherein the connection assembly (30) is a male buckle connector or a female buckle connector.

9. The device of claim 6, wherein
the FIR heating layer, the electrode insulating layer and the electrode layer form an FIR hearting electrode unit (26);
the connection assembly (30) includes:
a first connector (31A) installed on the FIR heating unit (26); and
a plurality of first positioning members (33A) provided on an upper surface of the conducting layer (25A);and
the conducting gel layer (25) including a conducting layer (25A) and a gel layer (27), with the first positioning members (33A) provided on the upper surface of the conducting layer (25A).

10. The device of claim 9, wherein the at least one electrode connecting end of the connection assembly is connected to a host in a wireless way; and
the host (10) includes:
a second connector (11) installed on a bottom of the host and connected to the first connector (31A); and
a plurality of second positioning members (12) provided on the bottom of the host and connected to the respective first positioning members (33A).
